# EUROPÄISCHE PATENTSCHRIFT

(11) **EP 2 682 606 B1**
(45) Veröffentlichungstag und Bekanntmachung des Hinweises auf die Patenterteilung: **04.03.2015**
(21) Anmeldenummer: 13174620.8
(22) Anmeldetag: 02.07.2013
(51) Int. Cl.: F04B 43/12

(54) **Multikonnektor und medizinisches Gerät zur extrakorporalen Blutbehandlung**
Multiconnector and medical device for extracorporeal blood treatment
Multiconnecteur et appareil médical pour le traitement extracorporel du sang

(30) Priorität: 03.07.2012 DE 102012105917
(43) Veröffentlichungstag der Anmeldung: 08.01.2014
(73) Patentinhaber: B. Braun Avitum AG, 34212 Melsungen (DE)
(72) Erfinder: Schäfer, Oliver, 36286 Neuenstein (DE); Möller, Dirk, 34326 Altmorschen (DE); Stenzel, Bruno, 26209 Hatten (DE)
(74) Vertreter: Winter, Brandl, Fürniss, Hübner, Röss, Kaiser, Polte - Partnerschaft mbB

(56) Entgegenhaltungen:
- DE-T2- 69 615 633
- US-A- 4 599 055
- US-B2- 8 047 819

## Beschreibung

Die Erfindung betrifft einen Multikonnektor zur Anbringung eines Schlauchsegments und zu- und abführenden Leitungen eines extrakorporalen Blutkreislaufs an einer Schlauchrollenpumpe.

Die Erfindung betrifft ferner ein medizinisches Gerät zur extrakorporalen Blutbehandlung, an dem ein solcher Multikonnektor anbringbar ist.

In medizinischen Geräten zur extrakorporalen Blutbehandlung (Dialyse) werden oftmals Schlauchrollenpumpen eingesetzt, welche das entnommene Blut des Patienten zu einem Dialysator und zum Patienten zurückfördern. Solche Schlauchrollenpumpen arbeiten peristaltisch, wobei ein schlaufenförmiges Schlauchsegment an einer entsprechend gebogenen Lauffläche des Pumpengehäuses anliegt. Ein innerhalb der Lauffläche liegender Rotor der Pumpe bewegt sich dann mit seinen Außenkanten entlang des Schlauchsegments, wobei es den Schlauch lokal eindrückt und so mit den elastischen Materialeigenschaften des Schlauchsegments eine Blutförderung durch das Schlauchsegment ermöglicht. Dafür wird das Blut dem Schlauchsegment über einen ersten Anschluss zugeführt und über einen weiteren Anschluss am anderen Ende des Schlauchsegments wieder abgeführt. Das Schlauchsegment bildet so beispielsweise zusammen mit den zu- und abführenden Leitungen und mehreren Luftfängern ein sogenanntes Überleitsystem, mit dem Blut des Patienten zu einem Dialysator und zum Patienten zurückgefördert wird.

Derartige Überleitsysteme werden vorzugsweise nach jeder Behandlung ausgetauscht und nicht wieder für andere Patienten verwendet. Ein benutztes Schlauchsegment muss somit aus der Pumpe entfernt werden, bevor ein neues Überleitsystem in das Gerät eingebracht wird. Um die Handhabung während dieses Ablegens und Aufrüstens des Überleitsystems zu erleichtern, ist es bekannt, an beiden Enden des Schlauchsegments jeweils einen Konnektor vorzusehen, der mit einer zu- bzw. abführenden Leitung verbunden werden kann. Allerdings dienen derartige Konnektoren lediglich als Verbindungsstücke und erfüllen keinerlei weitere Funktionen. Ein Nachteil solcher manueller Systeme mit Konnektoren besteht darin, dass es beim Einlegen in die Pumpe zu Verwechslungen kommen kann. Dieses Problem kann beispielsweise durch eine Farbcodierung und/oder eine geometrische Codierung gelöst werden.

Ferner sind beispielsweise aus US 4,599,055 B2 Multikonnektoren bekannt, die beide Anschlüsse für zu- bzw. abführende Leitungen in einem Bauteil vereinigen, das dann in eine Aufnahme des Pumpengehäuses eingebracht werden kann. Derartige Bauteile ermöglichen oftmals über ihre geometrische Form und ihre Materialeigenschaften ein einfaches und eindeutiges Einlegen des Pumpensegments in die Pumpe. Eine falsche Einbaulage wird dabei durch eine geometrische Codierung ausgeschlossen. Oftmals fehlt derartigen Systemen jedoch eine Betätigungsfläche zum Einsetzen des Systems in die Pumpe, was die Handhabung erschwert. So kann bei einer Schlauchpumpe gemäß US 4,599,055 B2 der Multikonnektor nur translatorisch an die Pumpe angebracht werden.

Darüber hinaus sind automatische Systeme bekannt, die das Ein- und Ausfädeln übernehmen und somit erleichtern sollen. Oftmals ist dabei zum Ausfädeln ein Aktuator zu betätigen, der das System beispielsweise über einen Linearantrieb aus seiner Therapieposition in eine Ausfädelposition bewegt. Hierzu kann es bei solchen Systemen erforderlich sein, einen Schalter/Knopf an dem medizinischen Gerät zu bedienen bzw. einen Softwarebutton auf einer Benutzeroberfläche zu berühren.

Zur Aufnahme eines Schlauchs innerhalb einer Rollenpumpe ohne Konnektoren beschreibt beispielsweise das Patent US 8,047,819 B2 Haltevorrichtungen, die lösbar am Pumpengehäuse angebracht sind. So können für verschiedene Schlauchgrößen und -arten unterschiedliche Haltevorrichtungen an der Pumpe montiert werden. Eine Haltevorrichtung weist dabei eine Klemmvorrichtung mit wenigstens einer schwenkbaren Klemmbacke auf, die eine halbkreisförmige Ausnehmung aufweist, so dass ein Schlauch in dieser halbkreisförmigen Ausnehmung und einer gegenüber liegenden, ebenfalls halbkreisförmigen Ausnehmung einer anderen Klemmbacke gehalten werden kann. Die Klemmbacken können auch mehrere dieser Ausnehmungen aufweisen, so dass mehrere Schläuche gleichzeitig aufgenommen werden können.

DE 696 15 633 T2 beschreibt ein System zum Laden und Entladen einer Schlauchrollenpumpe, wobei an einem Walzenkopf der Schlauchpumpe eine Nut vorgesehen ist, die das Wechseln einer in der Schlauchpumpe anzubringenden Schlauchschlaufe erleichtert.

Aufgabe der Erfindung ist es, einen Konnektor zur Anbringung eines Schlauchsegments und zu- und abführenden Leitungen eines extrakorporalen Blutkreislaufs an einer Schlauchrollenpumpe bereitzustellen, der eine einfache und sichere Handhabung des wenigstens durch das Schlauchsegment, den Konnektor und die zu- und abführenden Leitungen gebildeten Überleitsystems unterstützt.

Aufgabe der Erfindung ist es ferner, ein medizinisches Gerät zur extrakorporalen Blutbehandlung bereitzustellen, an welchem das Überleitsystem aus wenigstens dem Schlauchsegment, den zu- und abführenden Leitungen und dem erfindungsgemäßen Konnektor anbringbar ist.

Erfindungsgemäß wird diese Aufgabe durch einen Multikonnektor gemäß dem unabhängigen Anspruch 1 gelöst. Vorteilhafte Weiterbildungen des Multikonnektors ergeben sich aus den Unteransprüchen 2-10. Die Aufgabe wird ferner durch ein medizinisches Gerät zur extrakorporalen Blutbehandlung gemäß Anspruch 10 gelöst, wobei sich vorteilhafte Ausführungen dieses Geräts aus den Unteransprüchen 11-15 ergeben.

Der erfindungsgemäße Multikonnektor eignet sich zur Anbringung eines Schlauchsegments und zu- und abführenden Leitungen eines extrakorporalen Blutkreislaufs an einer Schlauchrollenpumpe, wobei der Multikonnektor wenigstens einen Grundkörper und zwei Konnektorelemente zur jeweiligen Verbindung der Enden des Schlauchsegments mit der zuführenden Leitung und der abführenden Leitung aufweist. Erfindungsgemäß sind die beiden Konnektorelemente an zwei gegenüber liegenden Seiten des Grundkörpers angebracht, wobei die Außenkontur eines ersten Konnektorelements wenigstens einen Gleitlagerabschnitt, insbesondere einen Abschnitt mit einer nach außen und gleichmäßig gekrümmten Außenkontur, vorzugsweise einen Hohlzylinderabschnitt, aufweist, der im Zusammenwirken mit einem entsprechenden Gleitlagerabschnitt am Pumpengehäuse der Schlauchrollenpumpe, insbesondere mit einer nach innen und gleichmäßig gekrümmten Ausnehmung im Pumpengehäuse der Schlauchrollenpumpe eine definierte Schwenkbewegung des Multikonnektors um eine Schwenkachse ermöglicht, und abseits des ersten Konnektorelements bzw. vom ersten Konnektorelement beabstandet sind Mittel zum Verrasten des Multikonnektors mit einem Pumpengehäuse der Schlauchrollenpumpe vorgesehen.

Durch diesen Aufbau des Multikonnektors ist es möglich, das erste Konnektorelement in eine Aufnahme an dem Pumpengehäuse einer Schlauchrollenpumpe einzubringen, wobei sich durch den zylindrischen Bereich des Konnektorelements ein Drehlager bilden lässt, so dass der Multikonnektor um die Zylinderachse des ersten Konnektorelements schwenkbar ist. Dies kann beim Vorgang des Einsetzens und Herausnehmens des Multikonnektors vorteilhaft genutzt werden, wobei die Mittel zum Verrasten des Multikonnektors am Pumpengehäuse abseits des ersten Konnektorelements vorgesehen sind. An dem erfindungsgemäßen Multikonnektor sind ferner Mittel vorgesehen, mit denen der Multikonnektor am Pumpengehäuse in einer Stellung gehalten werden kann, in der er über das erste Konnektorelement am Pumpengehäuse angebracht ist, jedoch nicht abseits des ersten Konnektorelements mit dem Pumpengehäuse verrastet ist. Es handelt sich somit um zusätzliche Verrastungsmittel, mit denen der Multikonnektor beispielsweise nach dem Herausschwenken am Drehlager in einer Ausfädelposition gehalten werden kann, ohne dass der Bediener zusätzliche Mechanismen bedienen muss, damit der Multikonnektor nicht wieder zurückschwenkt. Die grundlegenden Verrastungsmittel dienen dazu, den Multikonnektor in einer Therapiestellung mit dem Pumpengehäuse zu verrasten.

Der Multikonnektor und damit seine beiden Konnektoren können in einer ersten Ausführungsform der Erfindung fest mit dem Schlauchsegment und den zu- und abführenden Leitungen des extrakorporalen Blutkreislaufs verbunden sein. Dabei sind auf einer Seite des Multikonnektors die beiden Enden des Schlauchsegments, welches für die Pumpfunktion in die Schlauchrollenpumpe eingelegt wird, mit den Konnektorelementen verbunden, wodurch sich eine Schlauchschleife ergibt. Auf der anderen Seite des Multikonnektors ist jeweils eine zu- und eine abführende Leitung mit den Konnektorelementen verbunden. Diese Verbindungen der Schläuche mit dem Multikonnektor können durch Verklebung hergestellt werden.

In einer zweiten Ausführungsform der Erfindung weisen die Konnektorelemente des Multikonnektors jeweils wenigstens eine Aufnahme zur lösbaren Verbindung des Schlauchsegments und der zu- und abführenden Leitungen mit dem Multikonnektor auf. Der Multikonnektor bildet dann einen wiederverwendbaren Adapter, der beispielsweise mit einem Standard-Überleitungssystem verbindbar ist, wodurch die vorteilhafte Ausbildung des Multikonnektors auch für ein Standard-Überleitsystem genutzt werden kann. Beispielsweise ist ein Schlauchsegment über einzelne Standard-Konnektorelemente mit den zu- und abführenden Leitungen verbunden, und das Schlauchsegment kann dann für eine lösbare Verbindung in Aufnahmen innerhalb der Konnektorelemente des Multikonnektors eingedrückt werden. Anschließend kann der Multikonnektor zusammen mit dem daran angebrachten Überleitsystem an der Schlauchrollenpumpe angebracht werden.

Diese zweite Ausführungsform des erfindungsgemäßen Multikonnektors als Adapter für beliebig ausgebildete Überleitsysteme hat ferner den Vorteil, dass das Material des Multikonnektors nicht biokompatibel sein muss, da es keinen direkten Kontakt mit dem Blut des Patienten hat.

Die Mittel zum Verrasten des Multikonnektors in der Therapiestellung können beispielsweise an dem Grundkörper ausgebildet sein, welcher die beiden Konnektorelemente miteinander verbindet. Vorzugsweise sind die Mittel zum Verrasten des Multikonnektors in der Therapiestellung jedoch im Bereich des zweiten Konnektorelements vorgesehen oder werden durch das Konnektorelement selbst gebildet. Beispielsweise kann das zweite Konnektorelement ebenfalls wenigstens teilweise zylinderförmig ausgeformt sein, so dass es in eine entsprechend ausgeformte Aufnahme am Pumpengehäuse einrastbar ist, wenn die Geometrie und die Abmessungen entsprechend gewählt werden. Vorzugsweise sind beide Konnektorelemente zylinderförmig ausgeformt, wobei ihre Zylinderachsen parallel zueinander verlaufen. Hierdurch lässt sich erreichen, dass das erste Konnektorelement als Drehlager genutzt werden kann, um dessen Zylinderachse sich der Grundkörper mit dem zweiten Konnektorelement schwenken lässt, wobei das zweite Konnektorelement in eine gegenüber liegende Aufnahme eingedrückt werden kann, um eine Verrastung des Multikonnektors in der Therapiestellung zu erreichen.

Der verbindende Grundkörper zwischen den beiden Konnektorelementen ermöglicht eine gute Handhabung des Multikonnektors, da an ihm insbesondere eine Grifffläche ausgeformt sein kann, die von einem Bediener dazu genutzt werden kann, den Multikonnektor beim Einsetz- und Herausnahmevorgang zu bewegen. Hierbei ist auch eine Ein-Hand-Habung möglich. In einem Ausführungsbeispiel der Erfindung ist hierzu wenigstens eine Seitenfläche des Grundkörpers S-förmig ausgeführt. Durch diese Ausformung kann der Multikonnektor beispielsweise ergonomisch ausgebildet sein, um ihn einfach und sicher greifen zu können. Eine S-förmige oder sogar in sich verdrehte Form mit mehreren S-förmigen Flächen ermöglicht es dabei insbesondere, auch einen am Pumpengehäuse montierten Multikonnektor zu greifen und heraus zu schwenken, da so entsprechende Griffbereiche ausgebildet werden können, die auch im montierten Zustand zugänglich sind. Es ist somit kein Aktuator erforderlich, um den Multikonnektor aus der Therapiestellung herauszubewegen. Auch die Betätigung eines Schalters oder Knopfes, um den Ausfädelvorgang einzuleiten, ist nicht erforderlich.

Eine S-förmige oder anders ausgeformte Seitenfläche des Multikonnektors kann jedoch auch als geometrische Codierung genutzt werden, mit welcher der Multikonnektor gezielt an einem Pumpengehäuse ausgerichtet werden kann, wenn das Pumpengehäuse ebenfalls entsprechend ausgebildet ist.

Die zusätzlichen Verrastungsmittel können am Multikonnektor beispielsweise durch ein gebogenes Federelement realisiert werden, das im Bereich des ersten Konnektorelements am Grundkörper angebracht ist. Die Biegung des Federelements verläuft dabei im Wesentlichen entlang der Außenkontur des zylindrischen Bereiches des ersten Konnektorelements, wobei an dem Federelement wenigstens ein Rastelement angebracht ist. Das Rastelement kann wenigstens eine Einbuchtung oder Ausbuchtung am Federelement sein. Bei entsprechender Ausformung des Pumpengehäuses kann der Multikonnektor durch Verrastung am Pumpengehäuse so wenigstens in einer Schwenkposition gehalten werden.

Von der Erfindung umfasst ist ferner ein zugehöriges medizinisches Gerät zur extrakorporalen Blutbehandlung, umfassend wenigstens eine Schlauchrollenpumpe mit einem Pumpengehäuse, das eine gebogene Lauffläche und einen innerhalb der Lauffläche drehenden Rotor aufweist, wobei ein Schlauchsegment eines extrakorporalen Blutkreislaufs zwischen die Lauffläche und den Rotor einbringbar ist, und dem Schlauchsegment Blut über eine zuführende Leitung zuführbar ist, während Blut von dem Schlauchsegment über eine abführende Leitung abführbar ist. Der erfindungsgemäße Multikonnektor ist am Pumpengehäuse anbringbar, wozu am Pumpengehäuse eine Aufnahme vorgesehen ist, in welche der zylindrische Bereich des ersten Konnektorelements einbringbar ist, wodurch sich ein Drehlager bildet, durch das der Multikonnektor um die Zylinderachse des Konnektorelements schwenkbar ist. Darüber hinaus sind am Pumpengehäuse Mittel zum Verrasten eines Bereichs des Multikonnektors am Pumpengehäuse vorgesehen, der abseits des ersten Konnektorelements liegt.

Die Mittel zum Verrasten des Multikonnektors am Pumpengehäuse können beispielsweise durch eine Aufnahme am Pumpengehäuse gebildet werden, in welche das zweite Konnektorelement einrastbar ist. Wie bereits beschrieben, ist das zweite Konnektorelement dazu vorzugsweise zylinderförmig ausgebildet, so dass es in eine rinnenförmige Aufnahme eingebracht werden kann. Dabei sind die Geometrie und die Abmessungen dieser rinnenförmigen Aufnahme beispielsweise so gewählt, dass das zweite Konnektorelement über eine Kante der Aufnahme gedrückt werden muss, um in der Rinne der Aufnahme einzurasten. Dieser Vorgang findet bei der Montage des Multikonnektors statt, während das zweite Konnektorelement bei der Demontage über die Rastkante aus der Aufnahme gezogen werden muss, um die Verrastung wieder zu lösen. Der Multikonnektor und/oder das Pumpengehäuse sollten daher auf irgendeine Art elastisch ausgeformt sein, um die Verrastungsfunktion zu ermöglichen.

Ergänzend zu diesen Verrastungsmitteln abseits des Drehlagers sind am Pumpengehäuse weitere Mittel vorgesehen, um den Multikonnektor in wenigstens einer Schwenkposition des Drehlagers halten zu können. Dazu ist am Pumpengehäuse beispielsweise eine Rastnase zur Verrastung mit einem Federelement des Multikonnektors vorgesehen.

Ferner ist es erstrebenswert, den Multikonnektor in der Therapiestellung auch entlang der Zylinderachse des ersten Konnektorelements fixieren zu können. Dazu kann an dem Pumpengehäuse wenigstens eine Erhöhung ausgeformt sein, welche bei montiertem Multikonnektor an dem Grundkörper des Multikonnektors anliegt und so ein Verschieben verhindert, da der Multikonnektor in dieser Richtung formschlüssig mit dem Pumpengehäuse verbunden ist.

Typischerweise ist am Pumpengehäuse ein schwenkbarer Deckel angebracht, mit welchem wenigstens die Lauffläche und der Rotor der Pumpe abdeckbar sind, wobei der Deckel in einem Ausführungsbeispiel der Erfindung so ausgeformt ist, dass er bei Einbringung des ersten Konnektorelements in die zugehörige Aufnahme ein Gegenlager bildet. Die Geometrie des Deckels und des Multikonnektors sind somit aneinander angepasst.

Die Erfindung umfasst ferner ein solches medizinisches Gerät, bei dem ein erfindungsgemäßer Multikonnektor am Pumpengehäuse angebracht ist.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1: eine schematische Darstellung eines medizinischen Geräts zur extrakorporalen Blutbehandlung mit einer Blutpumpe;
- Fig. 2: eine schematische Darstellung einer Schlauchrollenpumpe mit eingelegtem Schlauchstück und einem Multikonnektor gemäß einer ersten Ausführungsform;
- Fig. 3a: eine erste schematische Seitenansicht einer Schlauchrollenpumpe nach Fig. 2;
- Fig. 3b: eine schematische Seitenansicht gemäß Fig. 3a mit ausgeschwenktem Multikonnektor;
- Fig. 4a: eine zweite schematische Seitenansicht einer Schlauchrollenpumpe nach Fig. 2;
- Fig. 4b: eine schematische Seitenansicht gemäß Fig. 4a mit ausgeschwenktem Multikonnektor;
- Fig. 5: eine schematische Seitenansicht gemäß Fig. 3a beim Entfernen des Multikonnektors.
- Fig. 6: eine schematische Darstellung einer Schlauchrollenpumpe mit eingelegtem Schlauchstück und einem als Adapter ausgeführten Multikonnektor gemäß einer zweiten Ausführungsform; und
- Fig. 7: eine schematische Seitenansicht einer Schlauchrollenpumpe nach Fig. 6.

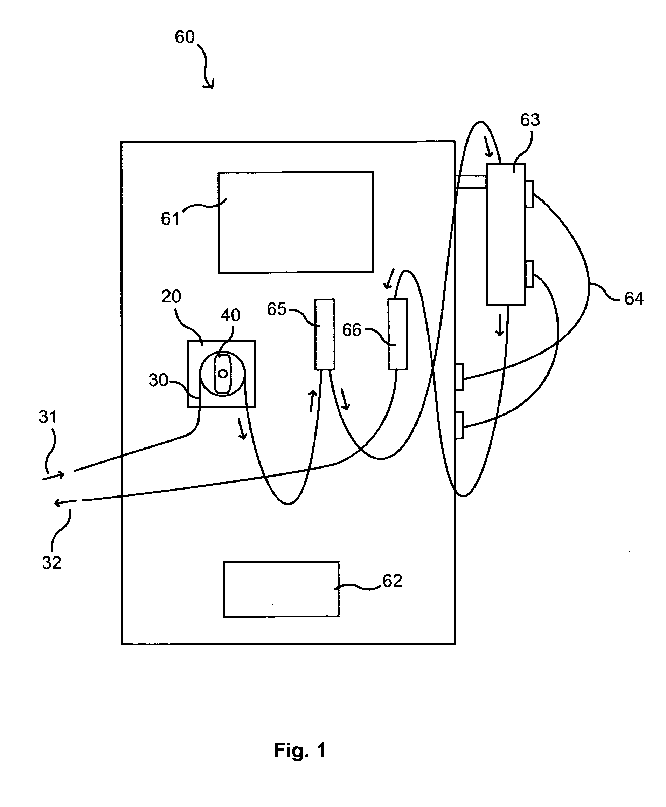

Fig. 1 zeigt eine schematische Darstellung der wesentlichen Grundkomponenten eines medizinischen Geräts bzw. Dialysegeräts 60 zur extrakorporalen Blutbehandlung mit einer Blutpumpe, wobei es sich bei der Blutpumpe um eine Schlauchrollenpumpe handelt. Die Schlauchrollenpumpe weist dabei ein Pumpengehäuse 20 auf, das typischerweise an der Frontseite des Dialysegeräts 60 angebracht ist.

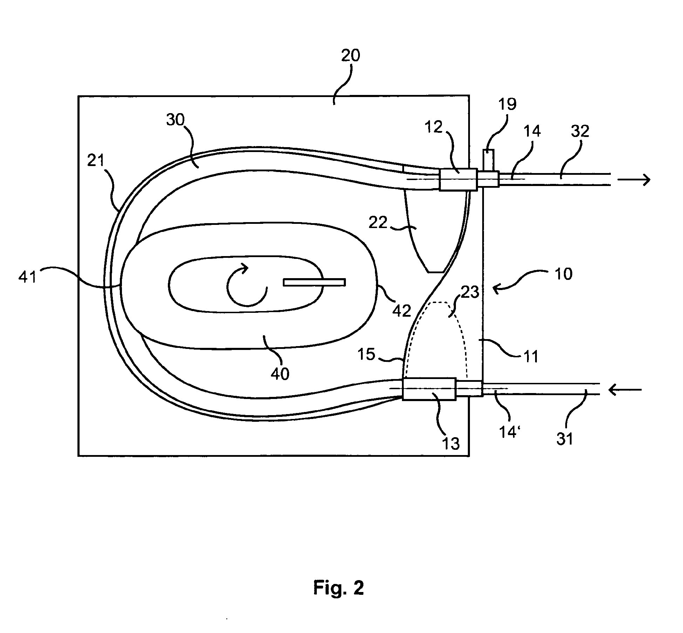

Dieser Schlauchrollenpumpe wird arterielles Blut 31 eines Patienten zugeführt und durch den extrakorporalen Blutkreislauf gefördert. Anschließend wird das Blut als venöses Blut 32 wieder zum Patienten zurückgeführt. Dabei wird das Blut mittels der Pumpe durch ein Überleitsystem gefördert, das an mehrere Komponenten des Dialysegeräts angeschlossen ist, wobei ein Schlauchsegment 30 des Überleitsystems in die Blutpumpe eingelegt ist und ein Rotor 40 das Blut peristaltisch durch dieses Schlauchsegment 30 fördert, wie es einer vergrößerten Ansicht der Fig. 2 zu entnehmen ist.

Nach Durchlaufen der Blutpumpe gelangt das Blut zum Dialysator 63, nachdem es vorzugsweise zuvor einen arteriellen Luftfänger 65 durchlaufen hat. Im Dialysator 63 wird das Blut durch Stoffaustausch mit einem Dialysat 64 gereinigt, welches dem Dialysator 63 zu- und abgeführt wird. Nach Durchlaufen des Dialysators 63 gelangt das Blut zu einem venösen Luftfänger 66 und wird dann dem Patienten zugeführt. Dieser Kreislauf des Bluts des Patienten ist in Fig. 1 durch Pfeile gekennzeichnet.

Die Einstellung von Parametern der Dialyse und die Überwachung der Therapie können über eine Anzeige-/Eingabeeinheit 61 erfolgen, die vorzugsweise als Touchscreen ausgebildet ist. Ferner weist das Dialysegerät 60 eine Steuer-/Regeleinheit 62 auf.

Fig. 2 stellt eine schematische Darstellung einer Schlauchrollenpumpe mit eingelegtem Schlauchsegment 30 und einem ersten Ausführungsbeispiel eines Multikonnektors10 dar. Die Schlauchrollenpumpe weist dabei das Pumpengehäuse 20 auf, das für den Bediener des Geräts leicht zugänglich ist, wobei das Pumpengehäuse 20 mit einem (in Fig. 2 nicht dargestellten) Deckel 50 abdeckbar ist, der über ein Scharnier zum Beispiel nach oben schwenkbar ist, um Zugriff auf das Schlauchsegment 30 zu erhalten.

In dem Pumpengehäuse 20 ist durch eine Vertiefung im Gehäuse eine kurvenförmige Lauffläche 21 ausgebildet, in welche das Schlauchsegment 30 schlaufenförmig eingelegt werden kann, so dass seine beiden Schlauchenden aus dem Gehäuse 20 herausragen. Dabei kann die Vertiefung mit einer Seitenfläche in dem Pumpengehäuse 20 ausgebildet sein, die im Wesentlichen gleichmäßig senkrecht zur Frontseite des Geräts verläuft, oder die Lauffläche 21 ist ungleichmäßig durch eine Seitenfläche der Vertiefung ausgeformt, die konkav oder sogar in sich verdreht ausgeformt ist.

Innerhalb der Lauffläche 21 ist ein Rotor 40 angebracht, der einen elliptischen Umfang hat, so dass er das Schlauchsegment 30 bei der Rotation an seinen Hauptscheiteln 41, 42 bzw. daran angebrachten Rollen leicht zusammendrücken kann. Durch die Drehung des Rotors 40 im Uhrzeigersinn bewegt sich der Bereich eines zusammengedrückten Schlauchsegments 30 ebenfalls im Uhrzeigersinn, bis sich der zugehörige Hauptscheitel 41 wieder vom Schlauchsegment 30 löst. In der Zeit hat der gegenüber liegende Hauptscheitel 42 jedoch bereits wieder Kontakt zu dem Schlauchsegment 30 aufgenommen, so dass Blut jeweils in dem Bereich des Schlauchsegments 30, in dem es von dem Rotor 40 zusammendrückt wird, peristaltisch vom Pumpeneingang zum Pumpenausgang gefördert wird.

An den beiden Enden des Schlauchsegments 30 ist der erfindungsgemäße Multikonnektor 10 angebracht, welcher eine Verbindung zu einer zuführenden Leitung 31 und einer abführenden Leitung 32 herstellt, durch welche somit Blut zu- und abgeführt wird, und über welchen das Schlauchsegement 30 und die beiden Leitungen 31 und 32 am Pumpengehäuse 20 lösbar befestigbar ist, wie weiter unten im Detail beschrieben wird. Diese Leitungen 31, 32 sind Teil des extrakorporalen Blutkreislaufs und sind mit verschiedenen Komponenten wie Luftfängern 65 und 66 und dem Dialysator 63 verbunden.

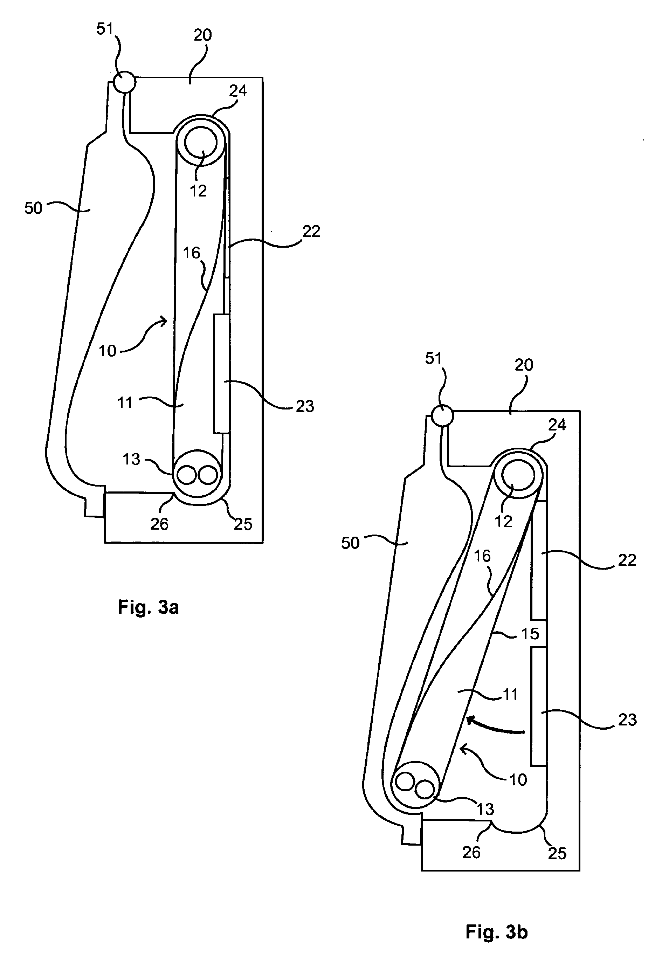

Der Multikonnektor 10 umfasst einen Grundkörper 11, der zwei gegenüber liegende Konnektorelemente 12 und 13 miteinander verbindet, die auch als Konnektoren bezeichnet werden können. Wenigstens das Konnektorelement 12 ist zylinder- bzw. hohlzylinderförmig ausgeführt, oder weist eine nach außen und gleichmäßig gekrümmte Außenfläche auf. Hierdurch kann dieses Konnektorelement 12 in eine entsprechend ausgeformte, insbesondere nach innen und gleichmäßig gekrümmte, Aufnahme 24 (siehe Fig. 3a) im Pumpengehäuse 20 werkzeuglos eingebracht bzw. eingelegt werden, wodurch sich ein Drehlager bzw. Gleitlager ergibt, um welches der Multikonnektor 10 bei der Montage und Demontage schwenkbar ist. Das zweite Konnektorelement 13 ist vorzugsweise ebenfalls zylinder- bzw. hohlzylinderförmig ausgeformt, wobei es sich auch um einen Zylinder mit abgestuften Außenflächen bzw. Rastflächen handeln kann. Die Zylinderachsen 14 und 14' der beiden Konnektorelemente 12, 13 verlaufen dabei parallel zueinander und quer zur Längserstreckung des Grundköpers 11 bzw. des Multikonnektors 10, so dass die beiden Leitungen 31, 32 ebenfalls parallel in die Pumpe bzw. aus der Pumpe geführt werden.

Der Multikonnektor 10 ist somit in Fig. 2 um den Drehpunkt am Konnektorelement 12 aus der Zeichenebene heraus schwenkbar. In der eingeschwenkten Stellung sind Mittel vorgesehen, mit denen der Multikonnektor 10 am Pumpengehäuse 20 verrastbar ist. Beispielsweise ist das zweite Konnektorelement 13 dazu so ausgeformt, dass es in eine Aufnahme 25 im Pumpengehäuse 20 einrastet, wenn es gegen das Pumpengehäuse 20 gedrückt wird. Es kann aber durch leichtes Ziehen auch wieder aus dieser Aufnahme 25 gelöst werden, wenn der Multikonnektor 10 aus der Zeichenebene der Fig. 2 herausgeschwenkt wird. Der Grundkörper 11 des Multikonnektors 10 ist daher vorzugsweise in sich leicht elastisch ausgebildet, so dass er ein Einrasten bzw. einen Formschluss in der Aufnahme 25 ermöglicht. Er kann jedoch auch relativ steif ausgeführt sein, wobei dann die Aufnahme 25 im Pumpengehäuse 20 elastisch ausgeführt wäre.

In der in Fig. 2 dargestellten Ansicht ist wenigstens eine linke Seitenfläche 15 des Grundkörpers 11 S-förmig ausgebildet. Diese Seitenfläche 15 stellt eine geometrische Codierung dar, wobei die Codierung jedoch auch andere geeignete Formen annehmen kann. Hierdurch ist es möglich, den Multikonnektor 10 gezielt am Pumpengehäuse 20 auszurichten, wobei er sich ferner gegen eine Bewegung entlang der Zylinderachsen 14, 14' am Pumpengehäuse 20 abstützt. Dazu ist am Pumpengehäuse 20 beispielsweise ein entsprechend ausgeformtes Stützelement 22 vorgesehen, das als Erhöhung am Pumpengehäuse 20 ausgeformt ist. An diesem Stützelement 22 liegt die Seitenfläche 15 des Grundkörpers 11 an. So kann der Multikonnektor 10 an dem Stützelement 22 ausgerichtet werden und gleichzeitig im Bereich des Pumpenausgangs nicht in die Pumpe hineingedrückt werden.

Der Grundkörper 11 ist vorzugsweise als kostengünstiger Einmalartikel ausgeführt und besteht aus einem Gebilde aus dreidimensionalen Flächen und/oder Streben, die nach außen offene Hohlräume ausbilden, so dass die Seitenfläche 15 an ihrer anderen Seite gleichzeitig an einem unterhalb des Multikonnektors 10 gestrichelt dargestellten Stützelement 23 anliegen kann. Dieses Stützelement 23 ist ebenfalls als Erhöhung am Pumpengehäuse 20 ausgebildet. Hierdurch kann der Multikonnektor 10 am Pumpeneingang nicht seitlich aus der Pumpe herausgezogen werden.

Weitere Flächen des Grundkörpers 11 können ebenfalls gebogen oder sogar in sich verdreht ausgebildet sein, wodurch beispielsweise an der rechten Seite des Grundkörpers 11 eine Grifffläche ausgebildet werden kann. An dieser kann der Multikonnektor 10 von einem Bediener gegriffen werden, um ihn dann erst in die obere Aufnahme 24 einzulegen und dann in die Zeichenebene hineinzuschwenken und in der unteren Aufnahme 25 zu verrasten. Um diesen Vorgang näher zu erläutern, zeigen die Figuren 3a und 3b eine erste Seitenansicht des Multikonnektors 10, wobei diese Ansicht in Fig. 2 einer Sicht von rechts entspricht.

Dabei zeigt Fig. 3a einen eingelegten Multikonnektor 10, dessen oberes Konnektorelement 12 in eine halbkreisförmige Aufnahme 24 im Pumpengehäuse 20 eingebracht ist. Durch die zylindrische Form des Konnektorelements 12 und der Aufnahme 24 ergibt sich an dieser Stelle ein Gleitlager mit einem Drehpunkt bzw. Drehachse, um den der Multikonnektor 10 schwenkbar ist. Dabei ist das untere Konnektorelement 13 in eine Aufnahme 25 eingerastet. Dies kann beispielsweise durch eine halbkreisförmige Aufnahme 25 erreicht werden, die eine als Rastkante fungierende Kante 26 (linke Kante der Aufnahme 25 in Fig. 3a und 3b) aufweist, die in den Schwenkbereich des Multikonnektors 10 um die Drehachse hineinragt, über die das Konnektorelement 13 erst gedrückt werden muss, um in die Aufnahme 25 zu gelangen. Die Verrastung kann jedoch auch an anderen Bauteilen erfolgen. Beispielsweise kann der Multikonnektor 10 durch eine entsprechende Geometrie auch innen am Stützelement 23 verrastet werden. Hierbei handelt es sich um die Therapiestellung des Multikonnektors 10 (Einfädelposition).

In dieser Ansicht ist auch erkennbar, dass das untere Konnektorelement 13 zwei Öffnungen zum Anschluss von Leitungen hat, die auf der anderen Seite in eine gemeinsame Röhre münden, die dann an das Schlauchsegment 30 in der Pumpe angeschlossen werden kann. So können verschiedene Leitungen angeschlossen werden. Beispielsweise kann eine Öffnung für die Blutzufuhr und ein anderer Anschluss für eine Druckmessung verwendet werden. Ferner kann am oberen Konnektorelement 12 ein weiteres Anschlussrohr 19 vorgesehen sein, das jedoch lediglich in der Fig. 2 dargestellt ist und senkrecht zur Zylinderachse 14 verläuft. An diesen Zusatzanschluss 19 kann ebenfalls eine weitere Leitung angeschlossen werden, wobei es sich beispielsweise um eine Heparinzufuhr handeln kann.

Darüber hinaus ist das Stützelement 23 erkennbar, durch welches der Multikonnektor 10 nicht aus der Pumpe herausgezogen werden kann, wobei das andere Stützelement 22 hinter dem Multikonnektor 10 angedeutet ist. Das Pumpengehäuse 20 wird ferner von einem Klappdeckel 50 abgedeckt, der um ein oberes Scharnier 51 schwenkbar ist. Gegen diesen Deckel 50 kann der Multikonnektor 10 über eine am Grundkörper 11 ausgebildete Grifffläche bei der Demontage gezogen werden, wie sie in Fig. 3b dargestellt ist. Bei dieser Stellung handelt es sich um eine definierte zweite Lage, die auch als Ausfädelposition bezeichnet werden kann.

Dazu kann der Multikonnektor 10 an seinem Grundkörper 11 gefasst und um den Drehpunkt in der oberen Aufnahme 24 geschwenkt werden, wie es durch den Pfeil dargestellt ist. Hierzu kann eine weitere S-förmige Fläche 16 des Grundkörpers 11 so ausgeformt sein, dass der Bediener den Multikonnektor 10 an dieser Fläche gut greifen kann. Dann muss die Verrastung der unteren Aufnahme 25 überwunden werden, indem das untere Konnektorelement 13 über die Rastkante 26 gezogen wird. Der geschlossene Deckel 50 der Pumpe dient dabei als Gegenlager und gleichzeitig als Endanschlag für den Multikonnektor 10 in der zweiten Endlage.

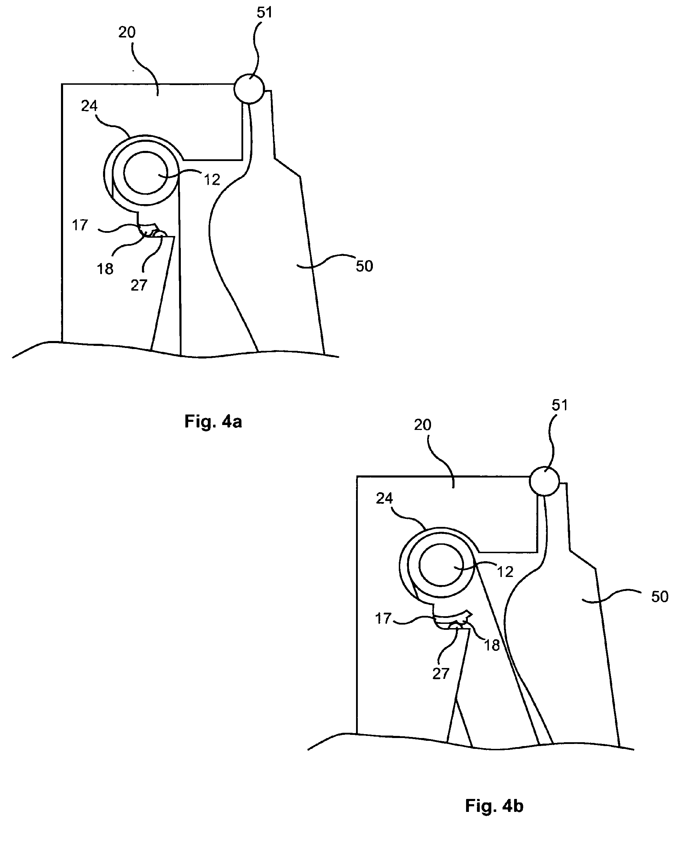

Damit der Multikonnektor 10 in der Ausfädelposition gehalten werden kann, ist beispielsweise am oberen Drehpunkt ein Federmechanismus vorgesehen. Die Funktionsweise dieses Federmechanismus ist den Figuren 4a und 4b zu entnehmen, die eine Sicht auf den Multikonnektor der Fig. 2 von links zeigen, wobei jedoch lediglich ein vergrößerter Ausschnitt des oberen Drehpunkts dargestellt ist. In der Fig. 4a ist der Multikonnektor 10 erneut in der Therapiestellung gezeigt, wobei auf dieser Seite des Grundkörpers 11 ein bogenförmiges Federelement 17 an dem Grundkörper 11 angebracht ist. Dieses Federelement 17 verläuft im Wesentlichen entlang der Außenkontur des Konnektorelements 12 und weist ein Rastelement in Form einer Erhöhung 18 auf. Diese Erhöhung 18 stößt in dieser Stellung des Multikonnektors 10 an eine Rastnase 27 an, die an dem Pumpengehäuse 20 ausgeformt ist. Der Multikonnektor 10 ist somit am oberen und am unteren Konnektorelement 12, 13 mit dem Pumpengehäuse 20 verrastet.

Beim Ausschwenken des Multikonnektors 10 aus der Therapiestellung in die Ausfädelposition muss dieser nicht nur aus der unteren, sondern auch aus der oberen Verrastung gedreht werden. Dies ist in Fig. 4b dargestellt, wobei ersichtlich ist, dass das obere Konnektorelement 12 beim Schwenken um den Drehpunkt mit dem Rastelement 18 über die Rastnase 27 am Pumpengehäuse 20 gezogen wurde. In dieser Stellung kann der Multikonnektor 10 gehalten werden, da das Rastelement 18 nun von der anderen Seite an der Rastnase 27 anliegt und alleine durch das Gewicht des Multikonnektors 10 nicht wieder zurückrutschen bzw. -zurückschwenken wird. Dabei dient der Pumpendeckel 50 vorzugsweise als Gegenlager.

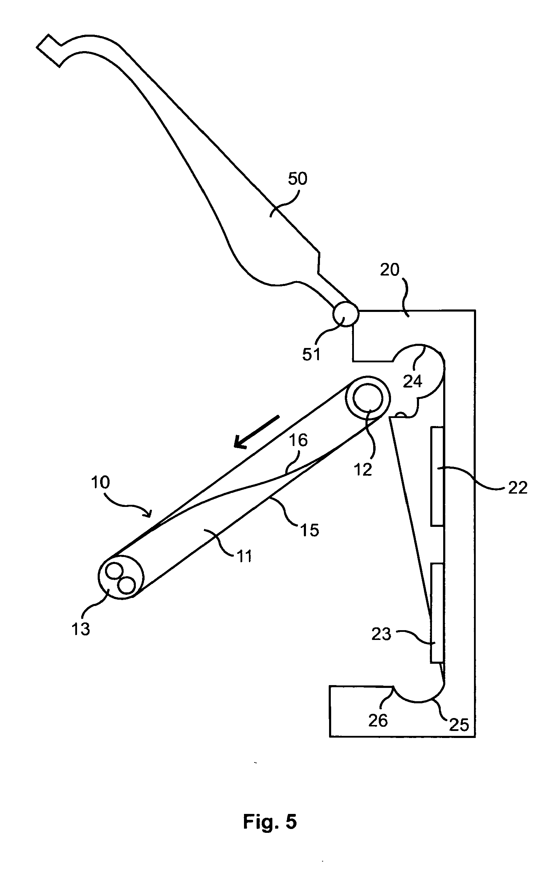

Fig. 5 zeigt die Entnahme eines Multikonnektors 10 aus dem Pumpengehäuse 20, wobei der Pumpendeckel 50 zuvor um das Scharnier 51 nach oben geschwenkt wurde, um den Multikonnektor 10 zu entnehmen. Es können jedoch auch andere Arten von Deckeln und Öffnungsmechanismen vorgesehen werden. Nach der Schwenkbewegung gemäß den Figuren 3b bzw. 4b kann der Multikonnektor 10 nun aus der oberen Aufnahme 24 herausgenommen werden, was durch einen Pfeil dargestellt ist.

Die Verrastungsvorgänge werden vorzugsweise dadurch erleichtert, dass der Grundkörper 11 durch seine Geometrie aus mehreren gebogenen Flächen elastisch ausgebildet ist. Als Material für den Grundkörper 11 und/oder die Konnektoren 12, 13 wird daher vorzugsweise ein Hartplastik eingesetzt, das eine solche Elastizität erlaubt.

Um den richtigen Einbau des Multikonnektors 10 am Pumpengehäuse 20 zu unterstützen, können Farbcodierungen vorgesehen sein. Je nach Ausführungsform kann ein falscher Einbau jedoch auch durch die spezielle Formgebung des Bauteils und des Pumpengehäuses 20 verhindert werden.

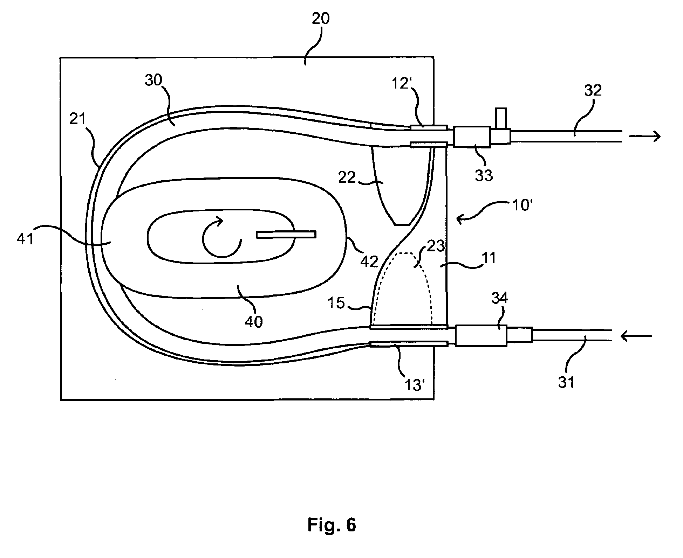

Fig. 6 zeigt eine schematische Darstellung einer Schlauchrollenpumpe mit eingelegtem Schlauchstück und einem Multikonnektor 10' gemäß einer zweiten Ausführungsform, bei welcher der Multikonnektor 10' als Adapter für ein Überleitsystem eingesetzt wird. Der Aufbau dieses Multikonnektors 10' entspricht im Wesentlichen dem Aufbau gemäß der ersten Ausführungsform, bei welcher das Schlauchsegment 30 und die zu- und abführenden Leitungen 31, 32 direkt und fest mit dem Multikonnektor 10 verbunden sind. Das anzuschließende Überleitsystem besteht erneut wenigstens aus einem Schlauchsegment 30 und den zu- und abführenden Leitungen 31 und 32, diese sind jedoch über einfache Verbindungselemente 33 und 34 mit dem Schlauchsegment 30 verbunden.

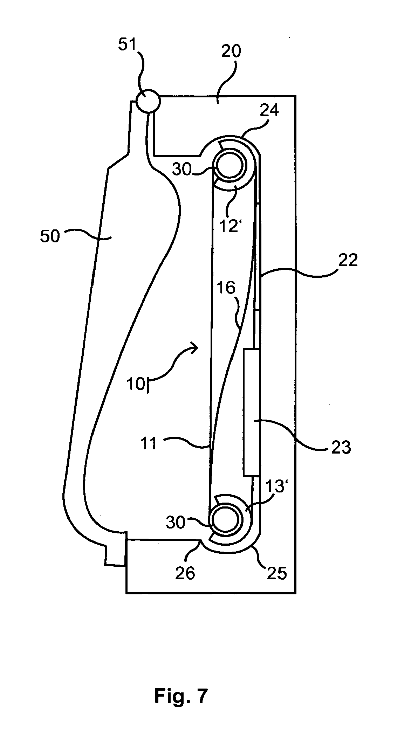

Um auch für ein solches Standard-Überleitsystem die Vorteile des erfindungsgemäßen Multikonnektors 10' nutzen zu können, sind die Konnektorelemente 12' und 13' des Multikonnektor-Adapters 10' so ausgeformt, dass der Schlauch des Schlauchsegments 30 lösbar an ihnen anbringbar ist. Dazu sind die Konnektorelemente 12' und 13' als Aufnahmen ausgeformt, was beispielsweise durch hohlzylindrische Konnektorelemente 12', 13' erreicht werden kann, die einen Längsschnitt aufweisen, in den das Schlauchsegment 30 eingedrückt werden kann. Diese Einbringung des Schlauchsegments 30 in die Konnektorelemente 12' und 13' ist auch der Seitenansicht der Fig. 7 zu entnehmen.

Nach der Therapie kann das Schlauchsegment 30 wieder aus dem Adapter 10' herausgezogen werden, und ein neues Schlauchsegment kann daran angebracht werden, so dass der Adapter ein wiederverwendbares Bauteil darstellt.

In einer alternativen Ausführungsform des Multikonnektors als Adapter sind die Konnektorelemente so ausgebildet, dass die Verbindungselemente 33 und 34 des anzubringenden Überleitsystems an den Konnektorelementen des Multikonnektors angebracht werden können, um das Überleitsystem mit dem Adapter zu verbinden. Auch hier bieten sich Rastverbindungen an. Auch eine Verbindung über die zu- und abführenden Leitungen oder eine Kombination mehrerer Verbindungsarten wäre möglich.

### Bezugszeichenliste

- 10,10': Multikonnektor
- 11: Grundkörper
- 12,12',13,13': Konnektorelement, Konnektor
- 14,14': Zylinderachse, Schwenkachse
- 15,16: Seitenfläche
- 17: Federelement
- 18: Rastelement
- 19: Zusatzanschluss
- 20: Pumpengehäuse
- 21: Lauffläche
- 22,23: Stützelement
- 24,25: Aufnahme
- 26: Rastkante
- 27: Rastnase
- 30: Pumpensegment, Schlauchsegment
- 31: Leitung, zuführend, arterielles Blut vom Patienten
- 32: Leitung, abführend, venöses Blut zum Patienten
- 33,34: Verbindungselement
- 40: Rotor
- 41,42: Hauptscheitel
- 50: Deckel, Pumpendeckel
- 51: Scharnier
- 60: Medizinisches Gerät zur extrakorporalen Blutbehandlung, Dialysegerät
- 61: Anzeige-/Eingabeeinheit, Touchscreen
- 62: Steuer-/Regelungseinheit
- 63: Dialysator
- 64: Dialysat
- 65: Arterieller Luftfänger
- 66: Venöser Luftfänger

## Patentansprüche

1. Multikonnektor (10; 10') zur Anbringung eines Schlauchsegments (30) und zu- und abführenden Leitungen (31, 32) eines extrakorporalen Blutkreislaufs an einer Schlauchrollenpumpe eines medizinischen Geräts, wobei
der Multikonnektor (10; 10') wenigstens einen Grundkörper (11) und zwei Konnektorelemente (12, 13; 12', 13') zur jeweiligen Verbindung der Enden des Schlauchsegments (30) mit der zuführenden Leitung (31) und der abführenden Leitung (32) aufweist,
die Konnektorelemente (12, 13; 12', 13') an zwei gegenüber liegenden Seiten des Grundkörpers (11) angebracht sind, wobei die Außenkontur eines ersten Konnektorelements (12; 12') wenigstens einen Gleitlagerabschnitt aufweist, der in einer im Pumpengehause der Schlauchrollenpumpe als Gleitlagerabschnitt entsprechend ausgebildeten Ausnehmung (24) werkzeuglos eingebracht ist, und wobei durch den Gleitlagerabschnitt des ersten Konnektorelements (12; 12') ein Drehlager (12, 24) gebildet ist, so dass der Multikonnektor (10, 10') um die Zylinderachse (14) des ersten Konnektorelements (12; 12') schwenkbar ist, und wobei
der Multikonnektor (10; 10') vom ersten Konnektorelement (12; 12') beabstandete Mittel (13, 13') zum Verrasten des Multikonnektors (10; 10') mit dem Pumpengehäuse (20) der Schlauchrollenpumpe aufweist,
**dadurch gekennzeichnet, dass**
am Multikonnektor (10; 10`) Mittel (17) vorgesehen sind, mit denen der Multikonnektor (10, 10`) am Pumpengehäuse (20) in einer Stellung gehalten wird, in der er über das erste Konnektorelement (12; 12') am Pumpengehäuse (20) geführt ist, jedoch nicht über die Mittel zum Verrasten am Pumpengehäuse (20) lösbar befestigt ist.

2. Multikonnektor nach Anspruch 1,
**dadurch gekennzeichnet, dass** die Konnektorelemente (12, 13) fest mit dem Schlauchsegment (30) und den zu- und abführenden Leitungen (31, 32) verbunden sind.

3. Multikonnektor nach Anspruch 1,
**dadurch gekennzeichnet, dass** die Konnektorelemente (12', 13') jeweils Mittel zur lösbaren Verbindung des Schlauchsegments (30) und der zu- und abführenden Leitungen (31, 32) mit dem Multikonnektor (10') aufweisen.

4. Multikonnektor nach einem oder mehreren der Ansprüche 1 bis 3,
**dadurch gekennzeichnet, dass** die Mittel zum Verrasten im Bereich des zweiten Konnektorelements (13, 13) vorgesehen sind oder durch das Konnektorelement (13, 13') selbst gebildet werden.

5. Multikonnektor nach einem oder mehreren der Ansprüche 1 bis 4,
**dadurch gekennzeichnet, dass** beide Konnektorelemente (12, 13; 12', 13') hohlzylinderförmig ausgeformt sind, wobei ihre Zylinderachsen (14; 14') parallel zueinander verlaufen.

6. Multikonnektor nach einem oder mehreren der Ansprüche 1 bis 5,
**dadurch gekennzeichnet, dass** am Grundkörper (11) zwischen den beiden Konnektorelementen (12, 13; 12', 13') eine Grifffläche ausgeformt ist.

7. Multikonnektor nach einem oder mehreren der Ansprüche 1 bis 6,
**dadurch gekennzeichnet, dass** der Grundkörper (11) wenigstens eine geometrische Codierung aufweist.

8. Multikonnektor nach Anspruch 1,
**dadurch gekennzeichnet, dass** in der Nähe des ersten Konnektorelements (12; 12') ein gebogenes Federelement (17) am Grundkörper (11) angebracht ist, dessen Biegung im Wesentlichen dem gekrümmten Außenkonturabschnitt des ersten Konnektorelements (12; 12') folgt, wobei an dem Federelement (17) wenigstens ein Rastelement (18) angebracht ist.

9. Multikonnektor nach Anspruch 8,
**dadurch gekennzeichnet, dass** das Rastelement wenigstens eine Einbuchtung oder eine Ausbuchtung (18) am Federelement (17) ist.

10. Medizinisches Gerät zur extrakorporalen Blutbehandlung, umfassend wenigstens eine Schlauchrollenpumpe mit einem Pumpengehäuse (20), das eine gebogene Lauffläche und einen innerhalb der Lauffläche (21) drehenden Rotor (40) aufweist, wobei ein Schlauchsegment (30) eines extrakorporalen Blutkreislaufs zwischen die Lauffläche (21) und den Rotor (40) eingebracht ist, und dem Schlauchsegment (30) Blut über eine zuführende Leitung (31) zugeführt ist, während Blut von dem Schlauchsegment (30) über eine abführende Leitung (32) abgeführt ist,
**dadurch gekennzeichnet, dass**
am Pumpengehäuse (20) ein Multikonnektor (10; 10') nach einem oder mehreren der Ansprüche 1 bis 9 angebracht ist, wozu am Pumpengehäuse (20) eine erste Aufnahme (24) vorgesehen ist, in welche der erste Gleitlagerabschnitt des ersten Konnektorelements (12; 12') angebracht ist, wodurch das Drehlager (12, 24) gebildet wird, durch das der Multikonnektor (10; 10') um die Zylinderachse (14) des ersten Konnektorelements schwenkbar ist, und
am Pumpengehäuse (20) ebenfalls Mittel zum Verrasten vorgesehen sind, die mit den am Multikonnektor (10; 10') vorgesehenen Mitteln zum Verrasten so zusammenwirken, dass der Multikonnektor (10; 10') zumindest in einer Relativlage am Pumpengehäuse lösbar befestigbar ist..

11. Medizinisches Gerät nach Anspruch 10,
**dadurch gekennzeichnet, dass** die Mittel zum Verrasten des Multikonnektors (10; 10') am Pumpengehäuse (20) durch eine zweite Aufnahme (25) am Pumpengehäuse (20) gebildet werden, in welche das zweite Konnektorelement (13; 13') einrastbar ist.

12. Medizinisches Gerät nach einem oder beiden der Ansprüche 10 und 11,
**dadurch gekennzeichnet, dass** am Pumpengehäuse (20) Mittel zur Fixierung des Multikonnektors (10; 10') entlang der Schwenkachse (14) des ersten Konnektorelements (12; 12') vorgesehen sind.

13. Medizinisches Gerät nach Anspruch 12,
**dadurch gekennzeichnet, dass** an dem Pumpengehäuse (20) wenigstens eine Erhöhung (27) ausgeformt ist, welche bei montiertem Multikonnektor (10; 10') an dem Grundkörper (11) des Multikonnektors (10; 10') anliegt.

14. Medizinisches Gerät nach einem oder mehreren der Ansprüche 10 bis 13,
**dadurch gekennzeichnet, dass** am Pumpengehäuse (20) eine Rastnase (27) zur Verrastung mit dem Federelement (17) des Multikonnektors (10; 10') gemäß Anspruch 8 vorgesehen ist, wodurch der Multikonnektor (10; 10') in wenigstens einer Schwenkposition des Drehlagers gehalten werden kann.

15. Medizinisches Gerät nach einem oder mehreren der Ansprüche 10 bis 14,
**dadurch gekennzeichnet, dass** am Pumpengehäuse (20) ein schwenkbarer Deckel (50) angebracht ist, mit welchem die Lauffläche (21) und der Rotor (40) abdeckbar sind, wobei der Deckel (50) so ausgeformt ist, dass er bei Einbringung des ersten Konnektorelements (12; 12') in die Aufnahme (24) ein Gegenlager bildet.

## Claims

1. A multi-connector (10; 10') for arranging a tubing segment (30) and feeding and discharging lines (31, 32) of an extracorporeal blood circulation on a tube roller pump of a medical device, wherein
the multi-connector (10; 10') comprising at least a base member (11) and two connector elements (12, 13; 12', 13') for connecting the respective ends of the tubing segment (30) to the feeding line (31) and the discharging line (32),
the connector elements (12, 13; 12', 13') are arranged on two opposed sides of the base member (11), wherein the outer contour of a first connector element (12; 12') includes at least one slide bearing portion which is inserted without a tool into a recess (24) correspondingly formed in the pump housing of the tube roller pump as slide bearing portion, and wherein a pivot bearing (12; 24) is formed by the slide bearing portion of the first connector element (12; 12'), so that the multi-connector (10; 10') is pivotable about the cylinder axis (14) of the first connector element (12; 12'), and wherein
the multi-connector (10; 10') includes means (13, 13') spaced apart from the first connector element (12; 12') for locking the multi-connector (10; 10') with the pump housing (20) of the tube roller pump,
**characterized in that**
at the multi-connector (10; 10') means (17) are provided by which the multi-connector (10; 10') is held at the pump housing (20) in a position in which it is guided at the pump housing (20) by the first connector element (12; 12'), but is not detachably fastened to the pump housing (20) via the means for locking.

2. The multi-connector according to claim 1,
**characterized in that** the connector elements (12, 13) are tightly connected to the tubing segment (30) and the feeding and discharging lines (31, 32).

3. The multi-connector according to claim 1,
**characterized in that** each of the connector elements (12', 13') includes means for detachably connecting the tubing segment (30) and the feeding and discharging lines (31, 32) to the multi-connector (10').

4. The multi-connector according to one or more of claims 1 to 3,
**characterized in that** the means for locking are provided in the area of the second connector element (13, 13') or are formed by the connector element (13, 13') itself.

5. The multi-connector according to one or more of claims 1 to 4,
**characterized in that** both connector elements (12, 13; 12', 13') have a hollow-cylindrical shape, with the cylinder axes (14; 14') thereof extending in parallel to each other.

6. The multi-connector according to one or more of claims 1 to 5,
**characterized in that** a gripping surface is formed at the base member (11) between the two connector elements (12, 13; 12', 13').

7. The multi-connector according to one or more of claims 1 to 6,
**characterized in that** the base member (11) has at least a geometrical encoding.

8. The multi-connector according to claim 1,
**characterized in that** in the vicinity of the first connector element (12; 12') a bent spring element (17), whose bending substantially follows the curved outer contour section of the first connector element (12; 12'), is arranged on the base member (11), wherein at least one stop element (18) is disposed at the spring element (17).

9. The multi-connector according to claim 8,
**characterized in that** the stop element is at least an indentation or a bulging (18) at the spring element (17).

10. A medical device for extracorporeal blood treatment comprising at least a tube roller pump including a pump housing (20) having a bent bearing surface and a rotor (40) rotating inside the bearing surface (21), wherein a tubing segment (30) of an extracorporeal blood circulation is introduced between the bearing surface (21) and the rotor (40) and blood is fed to the tubing segment (30) via a feeding line (31), while blood is discharged from the tubing segment (30) via a discharging line (32),
**characterized in that**
a multi-connector (10; 10') according to one or more of the claims 1 to 9 is arranged on the pump housing (20), for which purpose a first receiving portion (24) is provided at the pump housing (20) into which the first slide bearing portion of the first connector element (12, 12') is introduced, whereby the pivot bearing (12, 24) is formed by which the multi-connector (10; 10') is pivotable about the cylinder axis (14) of the first connector element, and
at the pump housing (20) means for locking are also provided which interact with the means for locking provided at the multi-connector (10; 10') so that the multi-connector (10; 10') can be detachably fastened to the pump housing at least in a relative position.

11. The medical device according to claim 10,
**characterized in that** the means for locking the multi-connector (10; 10') at the pump housing (20) are formed by a second receiving portion (25) at the pump housing (20) in which the second connector element (13; 13') is adapted to be engaged.

12. The medical device according to one or both of claims 10 and 11,
**characterized in that** means for fixing the multi-connector (10; 10') along the pivot axis (14) of the first connector element (12; 12') are provided at the pump housing (20).

13. The medical device according to claim 12,
**characterized in that** at the pump housing (20) at least one elevation (27) is formed which is adjacent to the base member (11) of the multi-connector (10; 10') when the multi-connector (10; 10') is mounted.

14. The medical device according to one or more of claims 10 to 13,
**characterized in that** a stop catch (27) for locking with the spring element (17) of the multi-connector (10; 10') according to claim 8 is provided at the pump housing (20), whereby the multi-connector (10; 10') can be held in at least a pivoting position of the pivot bearing.

15. The medical device according to one or more of claims 10 to 14,
**characterized in that** a pivotable pump cover (50) for covering the bearing surface (21) and the rotor (40) is arranged at the pump housing (20), the cover (50) being shaped so that it forms a counter-bearing when the first connector element (12; 12') is introduced into the receiving portion (24).

## Revendications

1. Multiconnecteur (10 ; 10') servant à mettre en place un tronçon de tuyau flexible (30) et des conduits d'arrivée et d'évacuation (31, 32) d'un circuit extracorporel sanguin au niveau d'une pompe à rouleau tubulaire d'un appareil médical,
le multiconnecteur (10 ; 10') présentant au moins un corps de base (11) et deux éléments de connecteur (12, 13; 12', 13') servant à relier respectivement les extrémités du tronçon du tuyau flexible (30) au conduit d'arrivée (31) et au conduit d'évacuation (32),
les éléments de connecteur (12, 13 ; 12', 13') étant installés au niveau de deux côtés se faisant face du corps de base (11), le contour extérieur d'un premier élément de connecteur (12 ; 12') présentant au moins un tronçon de palier à glissement, qui est mis en place sans outil dans un évidement (24) réalisé de manière correspondante dans le carter de pompe de la pompe à rouleau tubulaire sous la forme d'un tronçon de palier à glissement, et un palier rotatif (12, 24) étant formé par le tronçon de palier à glissement du premier élément de connecteur (12 ; 12') de sorte que le multiconnecteur (10, 10') peut être pivoté autour de l'axe de cylindre (14) du premier élément de connecteur (12 ; 12') et
le multiconnecteur (10 ; 10') présentant des moyens (13, 13') agencés à distance du premier élément de connecteur (12 ; 12') servant à enclencher le multiconnecteur (10 ; 10') avec le carter de pompe (20) de la pompe à rouleau tubulaire,
**caractérisé en ce**
**que** sont prévus au niveau du multiconnecteur (10; 10') des moyens (17), avec lesquels le multiconnecteur (10, 10') est maintenu au niveau du carter de pompe (20) dans une position donnée dans laquelle le multiconnecteur est guidé au niveau du carter de pompe (20) par l'intermédiaire du premier élément de connecteur (12 ; 12'), sans pour autant être fixé de manière amovible sur les moyens destinés à être enclenchés au niveau du carter de pompe (20).

2. Multiconnecteur selon la revendication 1,
**caractérisé en ce que** les éléments de connecteur (12, 13) sont reliés de manière solidaire au tronçon de tuyau flexible (30) et aux conduits d'arrivée et d'évacuation (31,32).

3. Multiconnecteur selon la revendication 1,
**caractérisé en ce que** les éléments de connecteur (12', 13') présentent respectivement des moyens servant à relier de manière amovible le tronçon de tuyau flexible (30) et les conduits d'arrivée et d'évacuation (31, 32) au multiconnecteur (10').

4. Multiconnecteur selon l'une quelconque ou plusieurs des revendications 1 à 3,
**caractérisé en ce que** les moyens sont prévus aux fins de l'enclenchement dans la zone du deuxième élément de connecteur (13, 13) ou sont formés par l'élément de connecteur (13, 13') lui-même.

5. Multiconnecteur selon l'une quelconque des revendications 1 à 4,
**caractérisé en ce que** deux éléments de connecteur (12, 13 ; 12', 13') sont formés de manière à présenter une forme de cylindre creux, leurs axes de cylindre (14 ; 14') s'étendant de manière parallèle les uns aux autres.

6. Multiconnecteur selon l'une quelconque des revendications précédentes 1 à 5,
**caractérisé en ce qu'**une surface de préhension est formée au niveau du corps de base (11) entre les deux éléments de connecteur (12, 13 ; 12', 13').

7. Multiconnecteur selon l'une quelconque ou plusieurs des revendications 1 à 6,
**caractérisé en ce que** le corps de base (11) présente au moins un codage à forme géométrique.

8. Multiconnecteur selon la revendication 1,
**caractérisé en ce qu'**un élément élastique (17) incurvé est installé au niveau du corps de base (11) à proximité du premier élément de connecteur (12 ; 12'), l'incurvation dudit élément élastique suivant essentiellement le tronçon de contour extérieur incurvé du premier élément de connecteur (12 ; 12'), au moins un élément d'enclenchement (18) étant installé au niveau de l'élément élastique (17).

9. Multiconnecteur selon la revendication 8,
**caractérisé en ce que** l'élément d'enclenchement est au moins une indentation ou une protubérance (18) au niveau de l'élément élastique (17).

10. Appareil médical servant au traitement extracorporel du sang, comprenant au moins une pompe à rouleau tubulaire pourvue d'un carter de pompe (20), qui présente une surface de roulement incurvée et un rotor (40) tournant à l'intérieur de la surface de roulement (21), un tronçon de tuyau flexible (30) d'un circuit extracorporel sanguin étant mis en place entre la surface de roulement (21) et le rotor (40), et du sang étant amené au tronçon de tuyau flexible (30) par l'intermédiaire d'un conduit d'arrivée (31), tandis que le sang est évacué du tronçon de tuyau flexible (30) par l'intermédiaire d'un conduit d'évacuation (32),
**caractérisé en ce**
**qu'**un multiconnecteur (10 ; 10') selon l'une quelconque ou plusieurs des revendications 1 à 9 est installé au niveau du carter de pompe (20), un premier logement (24) étant prévu à cet effet au niveau du carter de pompe (20), dans lequel le premier tronçon de palier à glissement du premier élément de connecteur (12 ; 12') est mis en place, ce qui permet de former le palier rotatif (12, 24), grâce auquel le multiconnecteur (10 ; 10') peut pivoter autour de l'axe de cylindre (14) du premier élément de connecteur, et
en ce sont prévus de la même manière au niveau du carter de pompe (20) des moyens d'enclenchement, qui coopèrent avec les moyens, prévus au niveau du multiconnecteur (10 ; 10'), servant à l'enclenchement, de sorte que le multiconnecteur (10 ; 10') peut être fixé de manière amovible au moins dans une position relative au niveau du carter de pompe.

11. Appareil médical selon la revendication 10,
**caractérisé en ce que** les moyens servant à enclencher le multiconnecteur (10 ; 10') au niveau du carter de pompe (20) sont formés par un deuxième logement (25) au niveau du carter de pompe (20), dans lequel le deuxième élément de connecteur (13 ; 13') peut être inséré par enclenchement.

12. Appareil médical selon l'un quelconque ou plusieurs des revendications 10 et 11,
**caractérisé en ce que** sont prévus au niveau du carter de pompe (20) des moyens servant à fixer le multiconnecteur (10 ; 10') le long de l'axe de pivotement (14) du premier élément de connecteur (12 ; 12').

13. Appareil médical selon la revendication 12,
**caractérisé en ce qu'**il est formé au niveau du carter de pompe (20), au moins une partie surélevée (27) laquelle repose, lorsque le multiconnecteur (10 ; 10') est monté, au niveau du corps de base (11) du multiconnecteur (10 ; 10').

14. Appareil médical selon l'une quelconque ou plusieurs des revendications 10 à 13,
**caractérisé en ce qu'**est prévu, au niveau du carter de pompe (20), un ergot d'enclenchement (27) destiné à l'enclenchement avec l'élément de ressort (17) du multiconnecteur (10 ; 10') selon la revendication 8, ce qui permet de maintenir le multiconnecteur (10 ; 10') dans au moins une position de pivotement du palier rotatif.

15. Appareil médical selon l'une quelconque ou plusieurs des revendications 10 à 14,
**caractérisé en ce qu'**est installé au niveau du carter de pompe (20) un couvercle (50) pouvant pivoter, par lequel la surface de roulement (21) et le rotor (40) peuvent être recouverts, le couvercle (50) étant formé de telle manière qu'il forme, lorsque le premier élément de connecteur (12 ; 12') est mis en place dans le logement (24), un contre-palier.
